# EUROPEAN PATENT APPLICATION

(11) **EP 2 042 210 A2**
(43) Date of publication of application: **01.04.2009**
(21) Application number: 08164906.3
(22) Date of filing: 23.09.2008
(51) Int. Cl.: A61M 25/00, A61M 5/315, A61M 5/28, A61M 5/50

(54) **Multiple Stage Fluid Delivery Device and Method of Use**

(30) Priority: 27.09.2007 US 995589 P
(71) Applicant: Tyco Healthcare Group LP, Mansfield, MA 02048 (US)
(72) Inventor: Chebator, Casey, Weymouth, Massachusetts MA 02093 (US); Sansoucy, Michael, Wrentham, Massachusetts MA 02093 (US)
(74) Representative: Gray, James

(57) **Abstract**

Multiple stage fluid delivery devices (10) are provided for dispensing a first fluid at a first flow rate and thereafter dispensing a second fluid at a second flow rate which is substantially lower than the first flow rate. The device (10) may be used to inject a fluid into a catheter to flush the catheter and to thereafter slowly deliver fluid into the catheter to maintain patency of the catheter.

## Description

This application claims priority from U.S. Provisional Application Serial No. 60/995,589, which was filed on September 27, 2007 and is incorporated herein in its entirety by reference.

### BACKGROUND

### 1. Technical Field

The present disclosure relates to multiple stage fluid delivery devices and their methods of use. More specifically, the present disclosure relates to a multiple stage fluid delivery device for delivering a first fluid at a first flow rate and a first or second fluid at a second reduced flow rate. The present disclosure also relates to a method of using the presently disclosed device for flushing and, thereafter, maintaining the patency of a catheter.

### 2. Background of Related Art

Intravenous or I.V. catheters which are inserted into a patient's vasculature, e.g., vein, to facilitate a variety of different medical procedures, including blood withdrawal, medication delivery, dialysis, etc., over an extended period of time are well known in the art. Such I.V. catheters minimize the pain and discomfort associated with multiple needle injections which may be required during a hospital stay.

One problem associated with I.V. catheters is that I.V. catheters are susceptible to clotting and may also lead to infection. More particularly, if blood stagnates within the catheter, the blood will eventually clot and occlude the catheter lumen. Further, stagnant blood provides a food source for planktonic bacteria which may form a biofilm and cause infection.

To overcome these problems, it is known in the art to flush the catheter lumen by injecting a fluid, e.g., saline, through the catheter lumen using a syringe. It is also known to inject a fluid using an elastomeric pump at a very low flow rate, e.g., 0.5ml/hour, into a catheter lumen over extended periods of time to maintain the patency of the catheter. Such a system is provided by I-Flow Corporation of Lake Forest, California, under the trademark KVO™ System.

A continuing need exists in the medical arts for a simple device which can provide an initial flush of a catheter and maintain the patency of the catheter over extended periods of time.

### SUMMARY

A method and device for flushing a catheter and for maintaining the patency of the catheter over an extended period of time are disclosed. The method comprises the steps of *i*) providing a fluid delivery device capable of delivering at least a first fluid at a first flow rate and a second fluid at a second lower flow rate; *ii*) delivering the first fluid to the catheter at the first flow rate with the fluid delivery device to flush the catheter; and *iii)* delivering the second fluid to the catheter at the second flow rate with the fluid delivery device to maintain the patency of the catheter by minimizing admittance of blood into the catheter.

In one embodiment, the first fluid and the second fluid are the same fluid, e.g., saline.

In one embodiment, the first flow rate is from about 0.1ml/sec to about 10ml/sec and second flow rate is from about 0.05ml/hr to about 10ml/hr. The first flow rate can be about 1ml/sec and the second flow rate can be about 0.5ml/hr. The first flow rate can be chosen by the user and may vary depending upon the technique of the user and the type of catheter being flushed.

In one embodiment, the step of providing a fluid delivery device includes providing a fluid delivery device having a housing defining a fluid outlet and a cavity, wherein the housing supports a primary plunger, a secondary plunger and a biasing member. The primary plunger and the secondary plunger are spaced to define first and second fluid reservoirs. The secondary plunger defines a throughbore to facilitate delivery of the second fluid to the fluid outlet.

In one embodiment, the throughbore of the secondary plunger is dimensioned to have a lower fluid flow rate than the fluid outlet of the housing.

The step of providing a fluid delivery device may include providing a fluid delivery device having a housing, a plunger, a biasing member and an actuator, wherein the housing has an open proximal end and a distal end defining a fluid outlet and the plunger is positioned within the housing and is urged by the biasing member towards the distal end of the housing. The plunger defines with a distal end of the housing a fluid reservoir and includes a distal extension having a channel formed therein. The plunger is movable from a retracted position to a partially advanced position to deliver fluid at the first flow rate from the fluid reservoir to the fluid outlet and from the partially advanced position to a fully advanced position to deliver fluid at the second fluid flow rate from the fluid reservoir through the plunger channel to the fluid outlet.

In one embodiment, the plunger channel includes a longitudinal portion and at least one transverse portion. The at least one transverse portion may include a plurality of transverse portions spaced along the distal extension. Each of the plurality of transverse portions can be in fluid communication with the longitudinal portion. In one embodiment, the distal extension is collapsible as the plunger is moved from the partially advanced position to the fully advanced position to sequentially close the respective transverse portions of the channel to progressively lower the fluid flow rate delivered through the fluid outlet.

### Brief Description Of The Drawings

Various embodiments of the presently disclosed multiple stage fluid delivery device and method of use are disclosed herein with reference to the drawings, wherein:
FIG. 1A is a side cross-sectional view of one embodiment of the presently disclosed multiple stage fluid delivery device prior to actuation;
FIG. 1B is a side cross-sectional view of the multiple stage fluid delivery device shown in FIG. 1A after a first stage of fluid delivery;
FIG. 1C is a side cross-sectional view of the multiple stage fluid delivery device shown in FIG. 1A during a second stage of fluid delivery;
FIG. 2A is a side cross-sectional view of a second embodiment of the presently disclosed multiple stage fluid delivery device prior to fluid delivery;
FIG. 2B is a side cross-sectional view of the multiple stage fluid delivery device shown in FIG. 2A after a first stage of fluid delivery;
FIG. 2C is a side cross-sectional view of the multiple stage fluid delivery device shown in FIG. 2A during a second stage of fluid delivery;
FIG. 3A is a side cross-sectional view of a third embodiment of the presently disclosed multiple stage fluid delivery device prior to fluid delivery;
FIG. 3B is a side cross-sectional view of the multiple stage fluid delivery device shown in FIG. 3A after a first stage of fluid delivery;
FIG. 3C is a side cross-sectional view of the multiple stage fluid delivery device shown in FIG. 3A after a second stage of fluid delivery;
FIG. 3D is a side cross-sectional view of the multiple stage fluid delivery device shown in FIG. 3A after multiple stages of fluid delivery;
FIG. 4A is a side cross-sectional view of a fourth embodiment of the presently disclosed multiple stage fluid delivery device prior to actuation; and
FIG. 4B is a side cross-sectional view of the multiple stage fluid delivery device shown in FIG. 4A during the second stage of fluid delivery.

### DETAILED DESCRIPTION OF EMBODIMENTS

Embodiments of the presently disclosed multiple stage fluid delivery device and its method of use will now be described in detail with reference to the drawings wherein like reference numerals designate identical or corresponding elements in each of the several views. In this description, the term proximally is generally used to indicate the relative nearness of a referenced item to a user of the device and the term distal is used to indicate the relative remoteness of a referenced item to a user of the device.

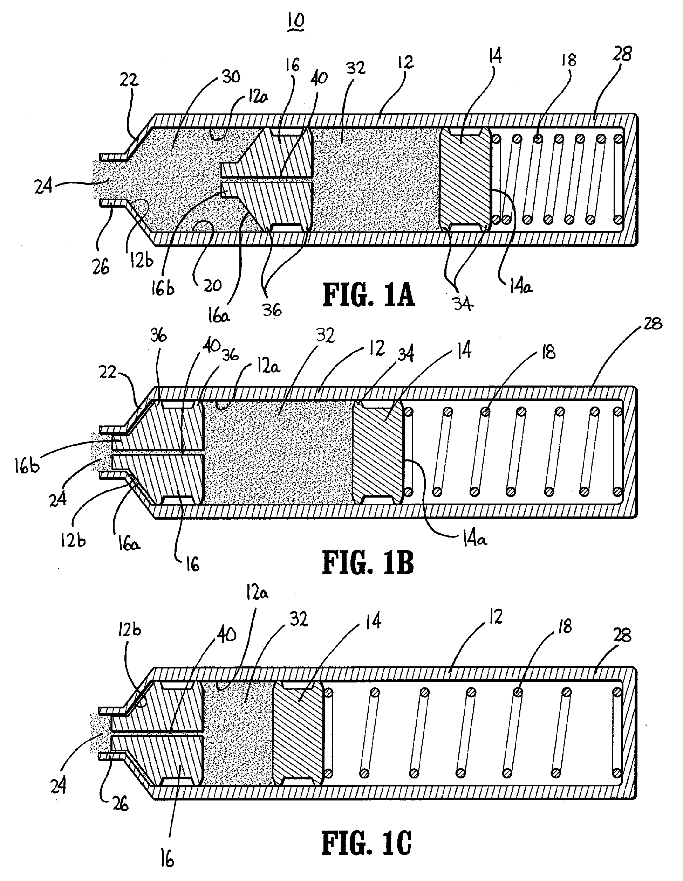

FIGS. 1A-1C illustrate one embodiment of the presently disclosed multiple stage fluid delivery device referenced generally as 10. Device 10 includes a housing 12, a primary plunger 14, a secondary plunger 16, and a biasing member 18. Housing 12 defines a fluid cavity 20 and includes a distal end 22 defining a fluid outlet 24. Distal end 22 includes engagement structure, e.g., a luer connector 26, for releasably engaging an I.V. catheter system. A proximal end 28 of housing 12 is illustrated as being enclosed. It is envisioned that proximal end 28 of housing 12 can be formed as a removable end cap (not shown) which can be threadably coupled or welded to the distal portion of housing 12 to confine biasing member 18 and the primary and secondary plungers 14 and 16 within cavity 20.

Biasing member 18 is positioned in the proximal end 28 of housing 12 and engages a proximal surface 14a of primary plunger 14. Secondary plunger 16 is spaced from primary plunger 14 and from the distal end of housing 12 to define spaced fluid reservoirs 30 and 32.

In the disclosed embodiment, each of plunger 14 and 16 includes a pair of spaced annular rings 34 and 36, respectively. Alternatively, additional rings may be provided about plungers 14 and 16. Each pair of rings 34 and 36 is sealingly engaged with an internal surface 12a of housing 12. As known in the art, internal surface 12a and/or plungers 14 and 16 can be coated with a lubricant to enhance slidable movement of plungers 14 and 16 through cavity 20. It is envisioned that a variety of plunger configurations can be used to achieve the objectives of the presently disclosed device as will be discussed in further detail below.

Secondary plunger 16 further includes a tapered distal face 16a and a distal extension 16b which are configured to substantially correspond to the shape of the distal end 12b of internal surface 12a of housing 12. As such, when secondary plunger 16 is fully advanced within cavity 20 of housing 12 (FIG. 1B), distal face 16a of plunger 16 is positioned adjacent distal end 12b of internal surface 12a of housing 12 and distal extension 16b of plunger 16 extends into fluid outlet 24. Such a plunger configuration allows for substantially all of the fluid in fluid reservoir 30 to be ejected from housing 12.

Secondary plunger 16 includes a small diameter through bore 40 which extends from a proximal surface of plunger 16 and through distal extension 16b. Small diameter bore 40 should be dimensioned to allow fluid reservoir 30 to empty prior to reservoir 32 upon actuation of biasing member 18, and thereafter, allow for a continuous flow of fluid from reservoir 32 to flow through bore 40 at a controlled lower flow rate.

Although not shown, a stop member is provided to retain primary plunger 14 in its retracted position (FIG. 1A) until medical personnel choose to dispense the contents of device 10. The stop member may comprise a tab which extends into housing 12 and engages plunger 14 or the distal end of biasing member 18 to prevent plunger 14 from pressurizing fluid within fluid reservoir 32. Alternatively, an opening could be provided in the proximal end of the housing for receiving an actuator. The actuator could be provided to engage and selectively release the primary plunger or to selectively load the biasing member.

Further, a cap (not shown) may be removably positioned over fluid outlet 24 to seal fluid outlet 24 prior to use.

In use, when biasing member 18 exerts a force on plunger 14, plunger 14 pressurizes the fluid in reservoir 32. This pressure is translated, via plunger 16, to the fluid in fluid reservoir 30. As discussed above, the dimensions of bore 40 through plunger 16 are selected such that the resistance to fluid flow is greater through bore 40 than from fluid outlet 24. As such, when biasing member 18 exerts a force on plunger 14, fluid from reservoir 30 is dispensed through outlet 24 at a first flow rate (FIG. 1B). When reservoir 30 empties and plunger 16 abuts the distal end 12b of internal surface 12a of housing 12, plunger 14 begins to move towards plunger 16 to dispense fluid from within fluid reservoir 32 through bore 40 and fluid outlet 24 at a second lower flow rate (FIG. 1C). In one embodiment, where device 10 is used to flush a catheter and thereafter to maintain the patency of the catheter, the dimensions of fluid outlet 24 and bore 40 can be selected so that the first flow rate is from about 0.1ml/sec to about 10ml/sec and the second flow rate can be from about 0.05ml/hr to about 10ml/hr. In one embodiment, the first flow rate is about 1ml/sec and the second flow rate is about 0.5ml/hr. It is noted that the first flow rate may be selectively varied based upon the type of catheter being flushed.

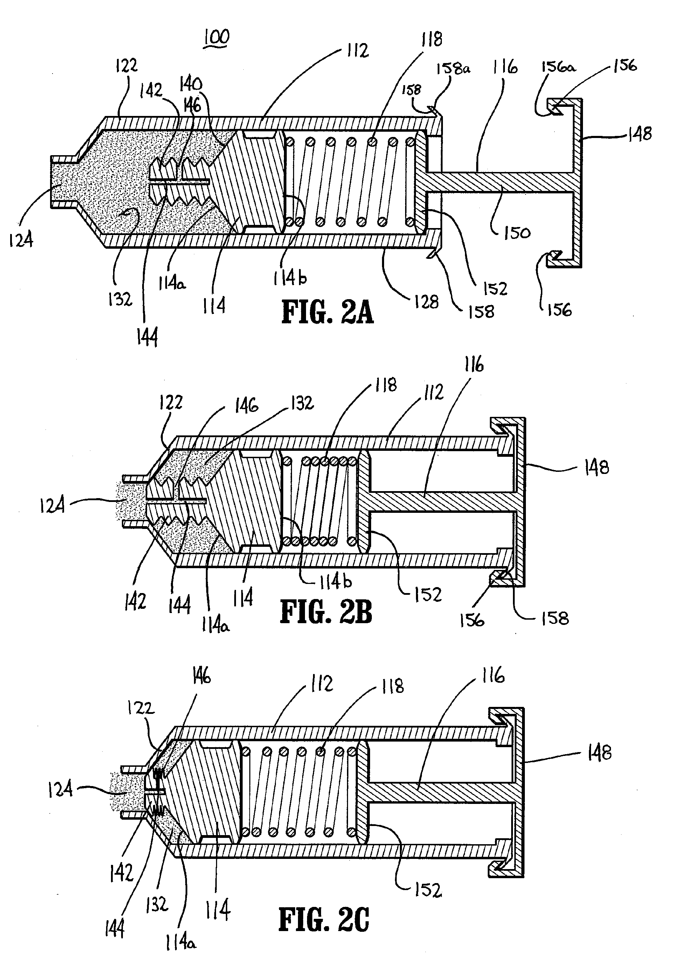

FIGS. 2A-2C illustrate an alternate embodiment of the presently disclosed multiple stage fluid delivery device shown generally as 100. Fluid delivery device 100 includes a housing 112, a plunger 114, an actuator 116 and a biasing member 118. Housing 112 defines a fluid reservoir 132 and includes a distal end 122 defining a fluid outlet 124. Plunger 114 is positioned within housing 112 such that fluid reservoir 132 is defined by the distal surface 114a of plunger 114 and the distal end 122 of housing 112. Biasing member 118 is positioned in the proximal end 128 of housing 112 between a proximal surface 114b of plunger 114 and a distal surface of actuator 116 as will be described in further detail below.

As discussed above, plunger 114 includes a distal surface 114a and a proximal surface 114b. Distal surface 114a is defined by a tapered or angled end wall 140 and a compressible distal extension 142. Distal extension 142 is in the form of a bellows-like structure. It is envisioned that distal extension 142 can be formed of the same material as the remaining portion of plunger 114 or of different materials as needed to achieve desired compression characteristics. A channel 144 extends through at least a portion of the length of distal extension 142. Channel 144 extends through the distal face of extension 142 and communicates with fluid reservoir 132 via a transverse channel 146. Distal extension 142 has an outer diameter which is greater than the diameter of fluid outlet 124. When plunger 114 is moved towards distal end 122 of housing 112 (FIG. 2B), distal extension 142 seals fluid outlet 124 as will be discussed in further detail below.

Actuator 116 includes a gripping member 148, a central shaft 150 and an abutment portion 152. Abutment portion 152 is configured to abut the proximal end of biasing member 118 such that upon movement of actuator 116 from a retracted position (FIG. 2A) to an advanced position (FIG. 2B), biasing member 118 is compressed between abutment portion 152 and proximal surface 114b of plunger 114. Gripping member 148 includes one or more locking members 156 which are configured to lockingly engage with locking structure 158 formed on a proximal end of housing 112 to retain actuator 116 in the advanced position (FIG. 2B). Locking member 156 can be formed in a substantially hooked configuration with a tapered engaging surface 156a and locking structure 158 can be formed as an angled extension 158a. When actuator 116 is moved to the advanced position, engaging surface 156a rides over angled extension 158a such that angled extension 158a is received within locking member 156 (FIG. 2B). In this position, biasing member 118, which may be a coil spring or the like, is compressed or loaded, to urge plunger 114 towards distal end 122 of housing 112. It is envisioned that movement of actuator 116 to the advanced position can be achieved by several means including the direct application of longitudinal force upon gripping member 148 and the translation of longitudinal force to actuator 116 such as by mechanical advantage systems known in the art, e.g. rotational to longitudinal translation.

As discussed above with respect to fluid delivery device 10, the distal end of housing 112 defining fluid outlet 124 includes engagement structure 126, e.g., luer, screw threads, etc., adapted to engage a medical device, e.g., a catheter assembly as is known in the art. Further, plunger 114 includes annular sealing rings 134 and 136 which sealingly engage an internal surface of housing 112. It is envisioned that other plunger configurations may be used.

In use, device 100 is secured to a medical device and actuator 116 is moved from the retracted position (FIG. 2A) to the advanced position (FIG. 2B). When this occurs, biasing member 118 is compressed or loaded and plunger 114 is urged towards distal end 122 of housing 112 to dispense fluid at a first flow rate through fluid outlet 124.

Referring to FIGS. 2B and 2C, when the distal end of distal extension 142 reaches distal end 122 of housing 112, distal extension 142 seals fluid outlet 124. As plunger 114 continues to be advanced by biasing member 118, fluid from reservoir 132 is forced to flow through transverse channel 146 and longitudinal channel 144 to exit fluid outlet 124. As discussed above, channels 144 and 146 are dimensioned to provide a second fluid flow rate which is substantially lower than the first fluid flow rate.

It is noted that channels 144 and 146 should be constructed so that they remain patent until fluid reservoir has been substantially emptied, even while distal extension 142 collapses (See FIG. 2C). It is envisioned that channels 144 and 146 may be reinforced with, for example, metallic or plastic inserts or the like, to ensure channels 144 and 146 remain patent.

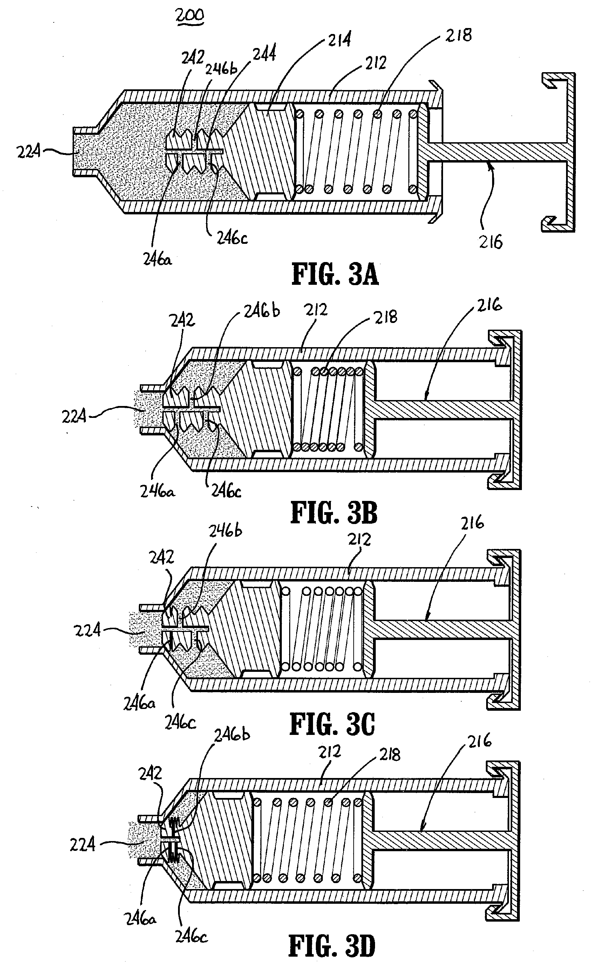

FIGS. 3A-3D disclose another embodiment of the presently disclosed multiple stage fluid delivery device 200. Device 200 is substantially the same as device 100 with a few distinctions which will be discussed below. Device 200 includes a plunger 214 having a distal extension 242 defining a longitudinal channel 244. Distal extension 242 further defines a plurality of spaced transverse channels 246a-c.

In use, device 200 functions just as device 100 functions in that as plunger 214 is moved distally by biasing member 218, fluid is dispensed from fluid reservoir 230 through outlet 224 at a first flow rate. However, except that when distal extension 242 seals fluid outlet 224 and begins to collapse, transverse passages 246a-c are sequentially sealed to provide a variable fluid flow rate which is continuously reduced. For example, as shown in FIG. 3B, fluid flows through passages 246a-c into longitudinal passage 244 at a second flow rate lower than the first flow rate. When distal extension 242 begins to collapse, passage 246a is sealed and fluid can only flow through passages 246b and 246c to provide a third lower flow rate. Thereafter, passages 246b and 246c are sequentially closed such that the fluid flow rate through outlet 224 is progressively decreased. Although three transverse passages are shown, it is envisioned that two or more passages may be provided, e.g., two, four, five, etc. It is noted that although not clear from the drawings, passages 246a-c and passage 244 must be dimensioned such that closure of passages results in a progressively reduced flow rate. In this respect, passage 244 should have a flow area equal to or greater than the combined flow area of transverse channels 246a-c.

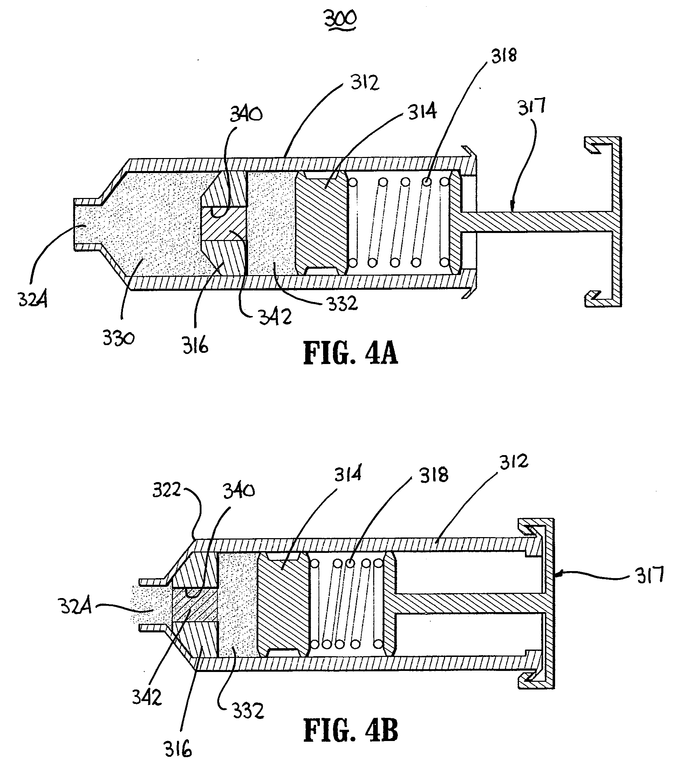

FIGS. 4A and 4B illustrate yet another embodiment of the presently disclosed multiple stage fluid delivery device shown generally as 300. Fluid delivery device 300 includes a housing 312, a primary plunger 314, a secondary plunger 316, an actuator 317, and a biasing member 318. Actuator 317 and biasing member 318 function substantially as described above with respect to actuator 116 and biasing member 118 and will not be discussed in further detail herein.

As illustrated, primary plunger 314 and secondary plunger 316 are positioned within housing 312 to define a fluid reservoir 330 between secondary plunger 316 and a distal end 322 of housing 312 and to define a fluid reservoir 332 between secondary plunger 316 and primary plunger 314. As discussed above with respect to fluid delivery devices 10, 100 and 200, housing 312 includes a fluid outlet 324 which is in fluid communication with fluid reservoir 330.

Secondary plunger 316 includes a throughbore 340 which is substantially filled with a porous material 342, e.g. In one embodiment, the porous material can comprise a polymer or a ceramic. If a polymer is employed, the polymer itself may be capable of allowing fluid flow therethrough. Examples of such polymers are hygroscopic materials, hydrogels, and so forth. In addition, polymer materials comprising open cell structures can be employed, wherein the structure of the flow restricting element will effect the rate of fluid flow therethrough. In such embodiments, open cell structures can be formed utilizing known manufacturing methods (e.g., blowing agents) for the open cell structure to allow fluid flow through the polymer. In yet another embodiment, a flow restricting element comprising a polymer can also be formed utilizing a sintering method, such as wherein a plurality of polymer particles are joined. In yet another embodiment, a plurality of polymer particles can be assembled within a structure, wherein the polymer particles are not specifically joined to one another, to form a flow restricting element.

The porous material can also comprise ceramics, such as metal oxides. Suitable metal oxides will have a suitable chemical stability such that the fluid flowing therethrough is not negatively affected by the ceramic. An example of a negative effect would be a catalytic reaction which would alter one or more components within the fluid. Another example of a negative effect would be exhibited if the ceramic induced one or more components of the fluid to bind to the ceramic. Examples of suitable metal oxides are silicon oxides, zinc oxides, cesium oxides, magnesium oxides, calcium oxides, aluminum oxides, as well as oxides comprising combinations comprising a plurality of metal ions, such as magnesium silicon oxides (e.g., Mg2SiO4), beryllium aluminum silicon oxides (Be3A12Si6O18), calcium silicon oxides (CsSi03), and so forth, as well as combinations comprising at least one of these ceramics.

Ceramic flow restricting elements by sintering, compression or assembly processes as well as by methods can be manufactured employing thermal spray coatings, such as air plasma spray (APS), vacuum plasma spray (VPS), high velocity oxy-fuel (HVOF), and deposition processes such as, for example, electron beam physical vapor deposition (EB PVD), plasma spray deposition, thermal spray deposition, or other appropriate deposition methods. The porous material 342 should be selected such that the resistance to fluid flow through porous material 342 is substantially greater than the resistance to fluid flow through fluid outlet 324.

In use, when actuator 317 is advanced distally and locked in its advanced position to load biasing member 318, primary piston 314 is advanced distally to pressurize fluid in reservoirs 332 and 330. Because the resistance to fluid flow through outlet 324 is less than through porous material 342, reservoir 330 empties at a first flow rate prior to reservoir 332. When reservoir 330 has emptied and secondary plunger 316 is positioned adjacent distal end 322 of housing 312, primary plunger 314, which is compressed by biasing member 318, forces fluid from within reservoir 332 to flow through porous material 342 and be dispensed through fluid outlet 324. As discussed above, the porosity of material 342 should be selected to provide a desired flow rate. Where device 300 is used to maintain the patency of a catheter, the flow rate through porous material 342 may be selected to be from about 0.05ml/hr. to about 10ml/hr.

It is also envisioned that other steps may be taken to control the fluid flow rate. For example, a biasing member having a variable spring force may be provided to adjust the fluid flow rate through a fluid outlet as the fluid is dispensed from the device,

It will be understood that various modifications may be made to the embodiments disclosed herein. For example, it is envisioned that the first and second fluids may be the same fluids, e.g., saline or different fluids. In addition, either or both of the first and second fluids may possess anti-microbial and/or anti-clotting properties, such as, a solution comprising EDTA. Therefore, the above description should not be construed as limiting, but merely as exemplifications of preferred embodiments. Those skilled in the art will envision other modifications within the scope and spirit of the claims appended hereto.

## Claims

1. A method for flushing a catheter and for maintaining the patency of the catheter over an extended period of time, the method comprising the following steps:
providing a fluid delivery device capable of delivering at least a first fluid at a first flow rate and a second fluid at a second lower flow rate;
delivering the first fluid to the catheter at the first flow rate with the fluid delivery device to flush the catheter; and
delivering the second fluid to the catheter at the second flow rate with the fluid delivery device to maintain the patency of the catheter by minimizing admittance of blood into the catheter.

2. A method according to Claim 1, wherein the first fluid and the second fluid are the same fluid.

3. A method according to Claim 1 or 2, wherein the first flow rate is from about 0.1ml/sec to about 10ml/sec and second flow rate is from about 0.05ml/hr to about 10ml/hr.

4. A method according to any preceding Claim, wherein the step of providing a fluid delivery device includes providing a fluid delivery device having a housing defining a fluid outlet and a cavity, the housing supporting a primary plunger, a secondary plunger and a biasing member, wherein the primary plunger and the secondary plunger are spaced to define first and second fluid reservoirs, the secondary plunger defining a throughbore to facilitate delivery of the second fluid to the fluid outlet at the second flow rate.

5. A method according to any of Claims 1 to 3, wherein the step of providing a fluid delivery device includes providing a fluid delivery device having a housing, a plunger, a biasing member and an actuator, the housing having an open proximal end and a distal end defining a fluid outlet, the plunger being positioned within the housing and being urged by the biasing member towards the distal end of the housing, the plunger defining with a distal end of the housing a fluid reservoir, the plunger including a distal extension having a channel formed therein, wherein the plunger is movable from a retracted position to a partially advanced position to deliver fluid at the first flow rate from the fluid reservoir to the fluid outlet and from the partially advanced position to a fully advanced position to deliver fluid at the second fluid flow rate from the fluid reservoir through the plunger channel to the fluid outlet.

6. The method according to Claim 5, wherein the plunger channel includes a longitudinal portion and at least one transverse portion.

7. The method according to Claim 6, wherein the at least one transverse portion includes a plurality of transverse portions spaced along the distal extension, each of the plurality of transverse portions being in fluid communication with the longitudinal portion.

8. The method according to Claim 6, wherein the distal extension is configured to be collapsible as the plunger is moved from the partially advanced position to the fully advanced position to sequentially close the respective transverse portions of the channel to progressively lower the fluid flow rate delivered through the fluid outlet.

9. A method according to Claim 4, wherein the throughbore of the secondary plunger is substantially filled with a porous material.

10. A multi-stage fluid delivery device comprising:
a housing defining a cavity and having a distal fluid outlet;
a primary plunger positioned within the cavity;
a secondary plunger positioned within the cavity at a location spaced from the primary plunger such that a first fluid reservoir having a first fluid is defined between a distal surface of the secondary plunger and a distal end of the housing and a second fluid reservoir having a second fluid is defined between a proximal surface of the secondary plunger and a distal surface of the primary plunger, the secondary plunger defining a throughbore which connects the first fluid reservoir to the second fluid reservoir;
a biasing member positioned to urge the primary plunger towards the distal end of the housing; and
a porous material positioned within the throughbore of the secondary plunger.

11. A multi-stage fluid delivery device according to Claim 10, wherein the porous material has a porosity which provides a substantially greater resistance to fluid flow than resistance to fluid flow through the distal fluid outlet of the housing, wherein when the biasing member urges the primary plunger towards the distal end of the housing, the first fluid is dispensed from the first reservoir through the fluid outlet at a first flow rate, and thereafter, the second fluid is dispensed from the second fluid reservoir through the porous material at a second fluid flow rate less than the first fluid flow rate.

12. A multiple stage fluid delivery device comprising:
a housing defining a cavity and having a distal fluid outlet;
a plunger supported within the cavity, the plunger having a distal extension having a channel defined therein, the distal end of the housing and a distal surface of the plunger defining a fluid reservoir, the plunger being movable from a retracted position to an advanced position to dispense a fluid from the fluid reservoir;
a biasing member positioned to urge the plunger towards the distal end of the housing; and
an actuator being movable to compress the biasing member to urge the plunger towards the distal end of the housing to dispense fluid through the fluid outlet from the fluid reservoir at a first flow rate;
wherein a distal end of the distal extension is dimensioned to seal the fluid outlet after the plunger has moved to a position between the retracted position and the advanced position such that further advancement of the plunger causes fluid to flow through the channel in the distal extension prior to being dispensed from the fluid outlet at a second fluid flow rate.

13. The multiple stage fluid delivery device according to Claim 12, wherein the channel is dimensioned such that the second flow rate is substantially less than the first flow rate.

14. The multiple stage fluid delivery device according to Claim 12 or claim 13, wherein the channel in the distal extension includes a longitudinal portion and at least one transverse portion in fluid communication with the longitudinal portion.

15. The multiple stage fluid delivery device according to Claim 14, wherein the at least one transverse portion includes a plurality of spaced transverse openings positioned along the distal extension.

16. The multiple stage fluid delivery device according to Claim 15, wherein the distal extension is collapsible to sequentially obstruct the transverse openings as the plunger is advanced towards the advanced position to progressively lower the flow rate of the fluid from the device.
